# EUROPÄISCHE PATENTSCHRIFT

(11) **EP 1 398 008 B1**
(45) Veröffentlichungstag und Bekanntmachung des Hinweises auf die Patenterteilung: **29.12.2010**
(21) Anmeldenummer: 03017383.5
(22) Anmeldetag: 31.07.2003
(51) Int. Cl.: A61F 2/44

(54) **Platzhalter für Wirbelkörper oder Bandscheiben**
Spacer for vertebrae or spinal discs
Ecateur pour vertèbres ou disques intervertébraux

(30) Priorität: 12.09.2002 DE 10242331
(43) Veröffentlichungstag der Anmeldung: 17.03.2004
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- EP-A- 0 176 728
- EP-A- 0 630 625
- EP-A- 1 346 709
- WO-A-01/68003
- WO-A-02/47586
- DE-C- 4 323 034
- US-A- 4 820 305
- US-A- 5 534 029

## Beschreibung

Die Erfindung betrifft einen Platzhalter für Wirbelkörper oder Bandscheiben.

Aus der EP 0 268 115 B ist ein Platzhalter der eingangs beschriebenen Art bekannt. Dieser dient insbesondere zum Ersetzen eines Wirbels. Dabei greifen die freien Enden des zylindermantelförmigen Elementes des Platzhalters in die jeweils benachbarten Wirbelkörper ein, so daß eine Fusion zwischen den beiden angrenzenden Wirbelkörpern und dem dazwischenliegenden Platzhalter erfolgt.

Aus der DE 43 23 034 ist ein Platzhalter der eingangs beschriebenen Art bekannt, der als Ersatz für eine entfernte Bandscheibe zwischen zwei benachbarte Wirbelkörper eingesetzt wird. Auch hier greift der Platzhalter mit seinen freien Enden in die benachbarten Wandungen der benachbarten Wirbelkörper ein, und es erfolgt eine Fusion zwischen den zwei angrenzenden Wirbelkörpern und dem die entfernte Bandscheibe ersetzenden Platzhalter.

Aus der EP 0 176 728 A1 ist eine Bandscheibenprothese bekannt, die aus einem annähernd kugelförmig, bzw. elliptisch im Querschnitt ausgebildeten Mittelteil aus Vollmaterial und zwei dazu kippbaren Abschlussplatten besteht.

Aus der WO 01/68003 A1 ist eine Bandscheibenprothese bekannt, die aus zwei kippbaren Abschlussplatten und einem ringförmigen Kern aus Vollmaterial besteht, der statt eines Lochs in der Mitte auf der Ober- und Unterseite eine konkave Vertiefung aufweist.

Aus der US 5 534 029 A ist ein Platzhalter für Wirbelkörper bekannt, der aus zwei kippbaren Abschlussplatten und einem quaderförmigen Mittelteil aus Vollmaterial besteht.

Aus der EP 0 630 625 A ist ein Platzhalter für Wirbelkörper bekannt, der einen mantelförmigen Mittelteil aufweist, bei dem die Abschlussplatten jedoch starr mit dem Mittelteil verbunden sind.

In der älteren nachveröffentlichten Europäischen Patentanmeldung EP 1 346 709 A2 ist ein Platzhalter für Wirbelkörper bekannt, der einen Hauptkörper, eine erste Endplatte und eine zweite Endplatte aufweist, wobei die zweite Endplatte auf einem unteren Ende des Hauptkörpers gesichert ist, wobei die zweite Endplatte an der Unterseite einen konvexen Vorsprung aufweist, der ausgebildet ist eine Kugel und eine Sockelanordnung mit dem zweiten konkaven Sockel zu bilden.

Aufgabe der Erfindung ist es, einen Platzhalter sowohl für Wirbelkörper als auch für Bandscheiben zu schaffen, der die Eigenschaften der oben als bekannt beschriebenen Platzhalter aufweist und mit dem eine bewegliche Verbindung zwischen dem Platzhalter und dem angrenzenden Wirbelkörper möglich ist.

Diese Aufgabe wird durch den im Patentanspruch 1 gekennzeichneten Platzhalter gelöst.

Die Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen:
- Figur 1: eine perspektivische Seitenansicht eines Platzhal- ters mit einem beweglichen Element auf einer Seite;
- Figur 2: eine perspektivische Seitenansicht eines Platzhal- ters mit beweglichen Elementen an jedem Ende;
- Figur 3: eine Schnittdarstellung durch eine erste Ausfüh- rungsform;
- Figur 4: eine Schnittdarstellung durch eine zweite Ausfüh- rungsform;
- Figur 5: eine Schnittdarstellung durch eine dritte Ausfüh- rungsform;
- Figur 6: eine Schnittdarstellung durch eine vierte Ausfüh- rungsform;
- Figur 7: eine Schnittdarstellung durch eine fünfte Ausfüh- rungsform;
- Figur 8: eine Schnittdarstellung durch eine sechste Ausfüh- rungsform;
- Figur 9: eine Draufsicht auf eine der Ausführungsformen nach den Figuren 5 bis 8, teilweise geschnitten;
- Figur 10: ein Detail aus Figur 9 mit vergrößertem Maßstab in einer ersten Stellung;
- Figur 11: das in Figur 10 gezeigte Detail in einer zweiten Stellung.

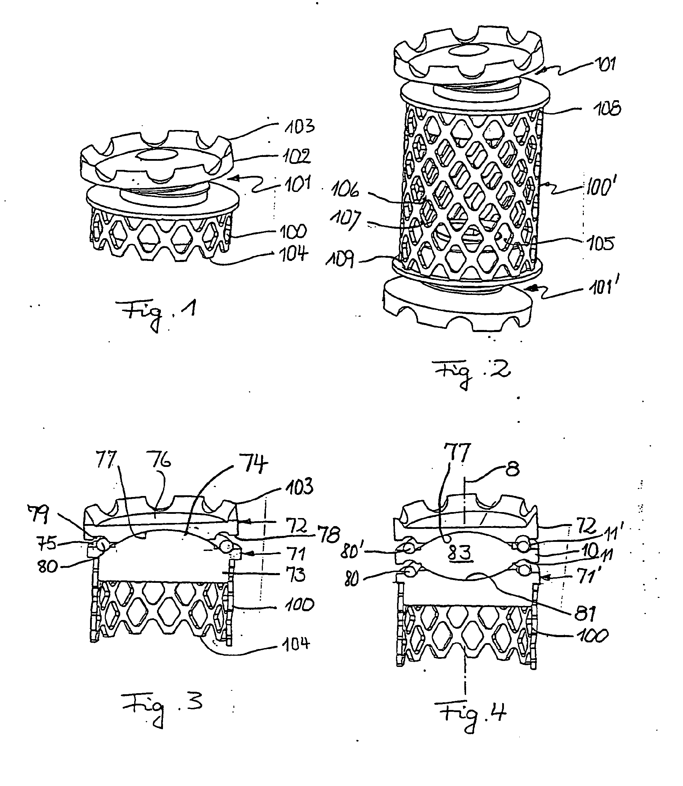

Figur 1 zeigt eine Ausführungsform, bei der ein rohrförmiger Abschnitt 100 so ausgebildet ist, daß er im Verhältnis zu seinem Durchmesser relativ kurz ist. An seinem einen Ende weist der rohrförmige Abschnitt 100 ein Element 101 auf, welches eine Deckplatte 102 aufweist, die relativ zu dem rohrförmigen Abschnitt beweglich ausgebildet ist. Sowohl die Deckplatte als auch der rohrförmige Abschnitt weisen an ihren freien Enden Zacken 103, 104 auf, die zum Eingreifen in die benachbarten Wirbelkörperendflächen vorgesehen sind. Ein solches Element mit kurzem rohrförmigen Abschnitt ist insbesondere für den Ersatz einer entfernten Bandscheibe bestimmt.

In Figur 2 ist der rohrförmige Abschnitt 101' länger ausgebildet und nicht nur an einem Ende wie in Figur 1, sondern auch an dem gegenüberliegenden Ende mit einem Element mit entsprechender Deckplatte verbunden. Die Ausbildung der beiden Elemente 101 und 101' ist vorzugsweise identisch. Diese Ausführungsform dient aufgrund der entsprechenden Länge des rohrförmigen Elementes 101' insbesondere als Ersatz für einen oder mehrere Wirbel. Wie aus den Figuren 1 und 2 ersichtlich ist, weisen die rohrförmigen Abschnitte 100, 100' Ausnehmungen 105 auf, um ein Einwachsen von Knochenmaterial zu erleichtern. In den Figuren ist eine besonders bevorzugte Ausführungsform gezeigt. Der rohrförmige Abschnitt 100 bzw. 100' ist als zylindrisch ausgebildeter Mantel geformt und weist sich mit ihrer Längsdiagonalen parallel zur Mantelachse erstreckende rautenförmige Ausnehmungen 105 auf. Jeweils benachbarte Reihen solcher Rauten sind in Richtung der Mantelachse um eine halbe Rautenhöhe versetzt. Dadurch wird ein Netz von sich unter einem spitzen Winkel schleifenden Bandstreifen 106, 107 gebildet, die unter jeweils gleich großen Winkeln gegen die Längsdiagonale der Rauten geneigt sind. Der obere Rand 108 und der untere Rand 109 erstrecken sich jeweils in einer Ebene senkrecht zu der Längsachse 2.

Im weiteren werden anhand der Figuren 3 bis 11 verschiedene Ausführungsformen der zusammen mit dem jeweiligen rohrförmigen Abschnitt einen Platzhalter bildenden Elemente beschrieben.

Wie aus den Figuren ersichtlich ist, weist jede Ausführungsform der Elemente eine Grundplatte und einer dieser gegenüberliegende Deckplatte auf.

Bei dem in Figur 3 gezeigten Ausführungsbeispiel ist die Grundplatte 71 als Zylinderelement ausgebildet, welches auf seiner der Deckplatte 72 zugewandten Seite eine Fläche mit einem ebenen Rand aufweist, deren Durchmesser gleich dem Durchmesser der Deckplatte 72 ist. Auf seiner der Deckplatte abgewandten Seite schließt sich ein zylindrischer Abschnitt 73 an, dessen Durchmesser ein wenig kleiner ist, so daß der darüberliegende Abschnitt mit größerem Durchmesser einen Anschlag bildet. Der Abschnitt 73 dient zum Aufnehmen eines rohrförmigen Abschnittes 100. Der rohrförmige Abschnitt 100 wird im Paßsitz auf den Abschnitt 73 aufgesetzt und weist an seinem freien Ende mit dem benachbarten Wirbelkörper in Eingriff bringbare Zacken 104 auf. Ferner weist der rohrförmige Abschnitt die oben beschriebenen Ausnehmungen 105 auf, die die Einwachsmöglichkeit wesentlich verbessern.

Die Grundplatte 71 weist auf ihrer der Deckplatte 72 zugewandten Seite eine zentral angeordnete konvexe Kontaktfläche 74 auf, die vorzugsweise sphärisch ausgebildet ist. Diese konkave Kontaktfläche ist von einem ebenen Rand umgeben. Die Deckplatte 72 weist eine Außenfläche 76 auf, die in dem gezeigten Ausführungsbeispiel eben ausgebildet ist und die an ihrem äußeren Rand sich nach außen zu der Außenfläche vertikal streckende Zacken 103 aufweist, die zum Eingreifen in eine benachbarte Wandung einer Wirbelkörperenplatte dienen. Auf der der Außenfläche 76 gegenüberliegenden Innenfläche weist die Deckplatte eine konkave Ausnehmung 77 auf, deren Ausbildung kongruent zur konvexen Kontaktfläche 74 ausgebildet ist. Angrenzend an die konkave Ausnehmung 77 und um diese herumlaufend erstreckt sich eine zu der Außenfläche 76 parallele Randzone 78. Wie weiter aus Figur 3 ersichtlich ist, weist die Randzone 78 auf der der Grundplatte zugewandten Unterseite benachbart zu der konkaven Ausnehmung 77 eine ringförmige Ausnehmung 79 auf. Diese weist in dem gezeigten Ausführungsbeispiel einen kreissegmentförmigen Querschnitt auf. Der dem gegenüberliegende ebene Rand 75 weist eine den gleichen Durchmesser aufweisende ringförmige Ausnehmung auf, die ebenfalls einen kreissegmentförmigen Querschnitt besitzt. In dem so gebildeten Paar der ringförmigen Ausnehmungen ist ein Ring angeordnet.

Bei der in Figur 4 gezeigten weiteren Ausführungsform stimmt die Ausbildung der Deckplatte mit der Deckplatte 72 vollständig überein. Die Grundplatte 71' unterscheidet sich von der zuvor beschriebenen Grundplatte 71 dadurch, daß anstelle der konvexen Kontaktfläche 74 eine konkave Kontaktfläche 81 vorgesehen ist, die in ihrer sphärischen Krümmung mit der konkaven Kontaktfläche 77 der Deckplatte 72 übereinstimmt. In allen übrigen Merkmalen stimmen die Grundplatte und der rohrförmige Abschnitt mit dem zuvor beschriebenen Ausführungsbeispiel überein.

Zwischen Grundplatte 71' und Deckplatte 72 liegt ein Kern 83. Dieser weist einen zur Symmetrieachse 8 symmetrisch angeordneten Zentralteil 9 auf, der die Form einer bikonvexen Linse aufweist und dessen jeweils konvexe Außenflächen die gleiche Krümmung und insbesondere sphärische Krümmung aufweisen, wie die damit zusammenwirkenden Kontaktflächen 74 und 77 der Grundplatte und der Deckplatte.

Wie die Figur weiter zeigt, weist auch der Kern 9 eine Randzone 10 auf, deren Außendurchmesser gleich dem Durchmesser von Grundplatte und Deckplatte ist. Die Randzone ist vorzugsweise so ausgebildet, daß die beiden der Grundplatte und der Deckplatte zugewandten Flächen zueinander und zu der Symmetrieebene des Kernes parallel ausgebildet sind. Auch die Randzone 10 weist auf beiden Seiten jeweils eine ringförmige Ausnehmung 11, 11' auf. Diese weisen den gleichen kreissegmentförmigen Querschnitt auf, wie die ringförmigen Ausnehmungen von Grundplatte und Deckplatte. Sowohl zwischen Grundplatte und Kern 9 als auch zwischen Kern und Deckplatte sind in den Rillen jeweils Ringe 80, 80' angeordnet.

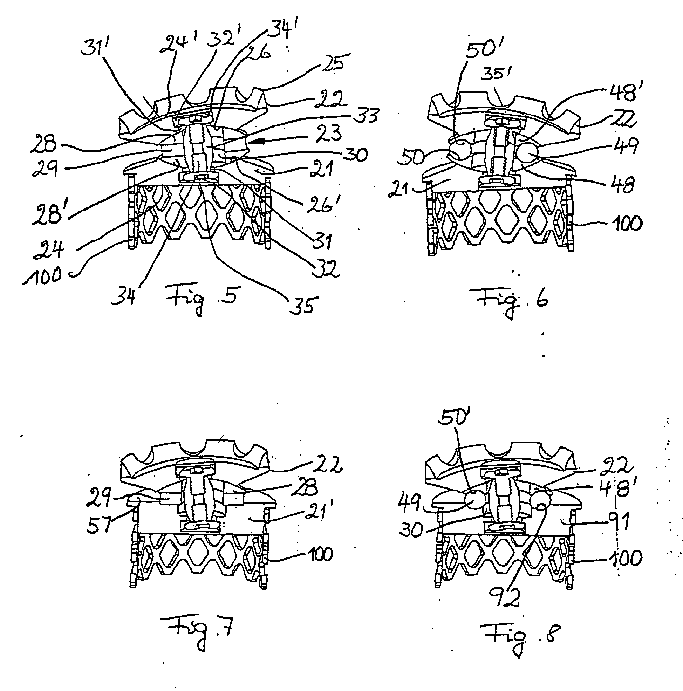

Bei den oben beschriebenen Ausführungsformen sind Grundplatte und Deckplatte jeweils aus einem biokompatiblen Material, insbesondere Stahl oder Titan hergestellt. Der Kern bei der in Fig. 4 gezeigten Ausführungsform ist aus einem körperverträglichen hochmolekularen Polyethylenkunststoff geformt. Die beiden Ringe 80, 80' sind aus einem körperverträglichen elastischen Kunststoff, beispielsweise Medical Grade Silikongummi gebildet. Bei den in den Figuren 5 und 6 gezeigten Ausführungsformen sind die elastische Zwischenschicht 29 beziehungsweise der Ring 48 ebenfalls aus einem körperverträglichen elastischen Kunststoff, beispielsweise Medical Grade Silikongummi hergestellt. Der rohrförmige Abschnitt 100 ist vorzugsweise aus Titan oder einem anderen körperverträglichen Material geformt.

Die in der Fig. 5 gezeigte dritte Ausführungsform weist wiederum eine Grundplatte 21, eine Deckplatte 22 und dazwischen einen Kern 23 auf.

Die Grundplatte 21 weist auf ihrer dem Kern 23 zugewandte Oberfläche symmetrisch zur Symmetrieachse 8 eine der konkaven Ausnehmung 81 entsprechende konkave Ausnehmung 26 auf. Es ist eine erste Randzone 27 vorgesehen, die anders als beim zweiten Ausführungsbeispiel aber nicht eben, sondern zur Außenseite der Grundplatte hin kegelstumpfförmig abfallend ausgebildet ist.

Die dem Kern 23 abgewandte Seite der Grundplatte 21 sowie die Verbindung mit dem rohrförmigen Abschnitt 100 ist genauso ausgebildet wie bei den vorher beschriebenen Ausführungsformen.

Die Deckplatte 22 weist wiederum nach außen hervorstehende Zacken 25 auf. Die Außenfläche 24' ist, wie am besten aus Fig. 5 ersichtlich ist, als konvexe kugelsegmentförmige Oberfläche ausgebildet, wobei die Krümmung der Oberfläche so gewählt ist, daß sie im wesentlichen einer typischen konkaven Krümmung einer damit in Kontakt zu bringenden Wirbelkörperendplattenfläche entspricht.

Die dem Kern 23 zugewandte Seite der Deckplatte 22 ist genauso ausgebildet wie die dem Kern zugewandte Seite der Grundplatte 21.

Der Kern 23 ist dreiteilig ausgebildet und besteht aus zwei mit ihren Planflächen einander zugewandten plan-konvexen Linsenkörpern 28, 28', zwischen denen eine plan-parallele Platte 29 angeordnet ist. Die Linsenkörper 28, 28' und die Platte 29 haben im wesentlichen den gleichen Durchmesser. Die Krümmung der konvexen Flächen der Linsenkörper entspricht der Krümmung der damit zusammenwirkenden konkaven Ausnehmungen 26, 26'.

Wie am besten aus Fig. 5 ersichtlich ist, weist der Kern 23 eine sich senkrecht zu seiner Symmetrieebene erstreckende und durch seinen Mittelpunkt gehende Bohrung 30 auf. An den entsprechenden Stellen weisen Grundplatte und Deckplatte sich entlang ihrer Symmetrieachsen erstreckende durchgehende Ausnehmungen 31, 31' auf. Auf den jeweiligen den Außenflächen 24, 24' zugewandten Seiten sind diese durch Senkbohrungen 32, 32' in ihrem Durchmesser erweitert. In der Bohrung 30 ist eine vorzugsweise aus einem körperverträglichen Kunststoff oder aus Metall gefertigte Verbindungshülse 33 vorgesehen, deren Durchmesser kleiner ist als der Durchmesser der Bohrung 30 und deren Länge größer als die Länge der Bohrung 30 ist, so daß die Verbindungshülse mit dem jeweiligen freien Ende in die Ausnehmung der benachbarten Platte eingreift. Wie aus Fig. 5 ersichtlich ist, ist die Hülse zu ihren Enden hin jeweils verjüngt ausgebildet. Von beiden Seiten ist jeweils durch die Ausnehmungen 31 geführt eine Schraube 34, 34' in die Verbindungshülse 33 eingeschraubt, wobei der Kopf der Schraube stets in der Senkbohrung anliegt. Die Senkbohrung ist ein wenig größer als der jeweilige Kopf. Die Schrauben werden so weit angezogen, daß Grund- und Deckplatte und Kern so miteinander verbunden sind, daß die aneinandergrenzenden Flächen ohne Spiel, aber zueinander beweglich gehalten sind.

Wie aus Fig. 5 ersichtlich ist, ist die Tiefe der Senkbohrungen 32, 32' etwas größer als die Dicke der Köpfe der Schrauben 34, 34'. Die Senkbohrungen sind an ihrem äußeren Ende jeweils durch Abdeckplatten 35 nach außen hin abgedeckt. Der Unterschied zwischen der Tiefe der Senkbohrungen 32, 32' und der Dicke der Köpfe der Schrauben 34, 34' ist so gewählt, daß die Köpfe beim federnden Zusammendrücken der Bandscheibenprothese gerade noch nicht an die Abdeckplatten 35 stoßen.

Die in Fig. 6 gezeigte Ausführungsform unterscheidet sich von der in Fig. 5 gezeigten Ausführungsform nur durch die Ausbildung des Kernes. Alle übrigen Teile stimmen mit der zuvor beschriebenen Ausführungsform überein.

Der Kern 43 weist wiederum zwei äußere plan-konvexe Linsenkörper 48, 48' auf, die mit ihren konvexen Flächen in gleicher Weise wie vorher beschrieben mit den Grund- und Deckplatten zusammenwirken. Auch die zentrale Bohrung und die Befestigung mittels der Verbindungshülse und den Schrauben stimmt identisch überein. Anders als bei dem vorherigen Ausführungsbeispiel ist anstelle der plan-parallelen Platte 29 ein elastischer Ring 49 vorgesehen. Zur Aufnahme und Führung des Ringes 49 weisen die einander zugewandten planen Flächen der Linsenkörper 48, 48' im Querschnitt kreisseigmentförmige ringförmige Ausnehmungen 50, 50' auf, in denen der Ring 49 gehalten ist.

Bei der in Fig. 7 gezeigten weiteren Ausführungsform stimmt die Deckplatte mit der in Fig. 5 beschriebenen Deckplatte überein.

Die Grundplatte 21' unterscheidet sich von der in den Figuren 5 und 6 gezeigten Grundplatte lediglich dadurch, daß die der Deckplatte 22 zugewandte Oberfläche 57 eben ausgebildet ist. In allen anderen Merkmalen stimmen die Grundplatte 21', der rohrförmige Abschnitt 100, sowie die Deckplatte 22 mit den anhand der Figuren 5 und 6 beschriebenen Ausführungsbeispielen überein.

Zwischen Grundplatte 21' und Deckplatte 22 ist wiederum ein Kern vorgesehen, der auf seiner der Deckplatte 22 zugewandten Seite einen plan-konvexen linsenförmigen Abschnitt 28 aufweist, der mit dem entsprechenden Abschnitt von Ausführungsform gemäß Figur 5 übereinstimmt. Zwischem diesem und der ebenen Fläche 57 der Grundplatte 21' ist eine plan-parallele Platte 29 vorgesehen. Die Materialien von Grundplatte und Deckplatte und linsenförmigen Körper 28 sind identisch mit den zuvor beschriebenen Ausführungsbeispielen. Die Materialwahl der plan-parallelen Platten 29 stimmt mit der der plan-parallelen Platten 29 aus Fig. 5 überein.

Die Grundplatte 91 unterscheidet sich gegenüber der in Fig. 7 beschriebenen Grundplatte 21' dadurch, daß sie unmittelbar um die Bohrung 30 herum eine ringförmige Ausnehmung mit einem kreissegmentförmigen Querschnitt aufweist. Anstelle des Kernes des zuvor beschriebenen Ausführungsbeispieles ist ein plan-konvexer Linsenkörper 48' vorgesehen, der mit seiner konvexen phärischen Oberfläche mit der Kontaktfläche der Deckplatte 22 zusammenwirkt und der auf seiner der Grundplatte zugewandten ebenen Fläche eine ringförmige Ausnehmung 50' aufweist, die in ihren Abmessungen denen der Ausnehmung 92 entspricht. Es ist ein Ring 49 vorgesehen, der in diesen beiden ringförmigen Ausnehmungen gelagert ist.

Die Materialien von Grundplatte und Deckplatte und Linsenkörper des Kernes stimmen mit denen des zuvor beschriebenen Ausführungsbeispieles überein. Der Ring 49 stimmt in seiner Materialauswahl mit dem Material der plan-parallelen Platte 29 des zuvor beschriebenen Ausführungsbeispieles überein.

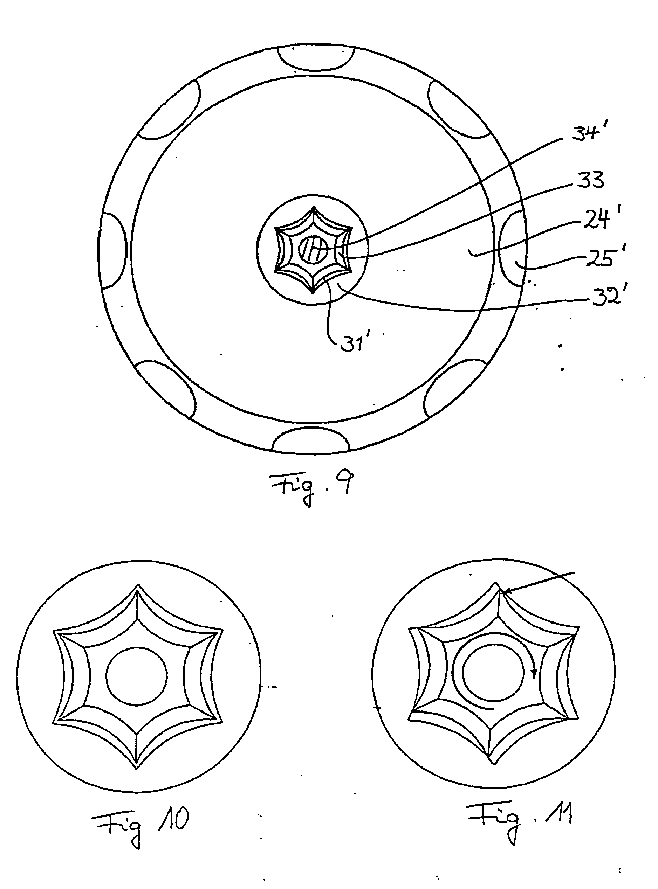

In Fig. 9 ist eine Draufsicht auf eine Deckplatte der in den Fig. 5 bis 8 beschriebenen Ausführungsformen gezeigt, wobei die Abdeckplatte 35' und der Kopf der Schraube 34' weggelassen sind.

Aus Fig. 9 ist zu ersehen, daß die jeweilige Hülse 33 an ihren jeweils verjüngt abgeschrägten Enden sechskantig ausgebildet ist, wobei die Flächen zwischen den sechs Ecken jeweils hohlkehlenartig ausgebildet sind. Die diesen sechskantigen Abschnitt aufnehmende jeweilige Ausnehmung 31' ist ebenfalls sechskantig ausgebildet, wobei der jeweilige Durchmesser durch zwei gegenüberliegende Ecken jeweils um ein vorbestimmtes Maß wenig größer als der entsprechende Durchmesser der Verbindungshülse an dieser Stelle ist. Die Flächen zwischen jeweils zwei Ecken sind zur Mitte der Ausnehmung hin bauchig ausgebildet, wobei der Radius der bauchigen Krümmung jeweils um ein vorbestimmtes Maß wenig größer als der Radius der Hohlkehlen ist.

Wie in Fig. 10 und Fig. 11 gezeigt ist, kann somit eine Drehung um ein durch die Größenunterschiede vorbestimmtes Maß zwischen Hülse und Deckplatte bzw. Hülse und Grundplatte erfolgen. Damit wird eine Begrenzung der Drehung auf einen vorbestimmten Winkel erreicht.

Bei allen gezeigten Ausführungsformen können die Außenflächen von Grund- und Deckfläche rauh ausgebildet sein, um eine Verbesserung des Einwachsens zu erreichen.

Bei allen oben beschriebenen Ausführungsbeispielen können die aneinandergrenzenden und eine Relativbewegung zueinander ausführenden Flächen mit entsprechendem Material als Gleitpaarung beschichtet sein. Dafür kommen insbesondere Keramikschichten oder auch Polyethylenbeschichtungen oder auch entsprechende Metallegierungen in Frage.

Bei den oben beschriebenen Ausführungsformen sind jeweils aneinandergrenzende und zusammenwirkende konkave und konvexe sphärische Flächen beschrieben. Dabei hat jeweils der Kern die konvexen Flächen und die Deckplatte und die Grundplatte haben zugehörige konkave sphärische Flächen. Nach einer abgewandelten Ausführungsform können die Flächenformen jeweils umgekehrt sein. Das heißt, der Kern kann als bikonkaver Linsenkörper oder als plan-konkaver Linsenkörper ausgebildet sein und die zugehörige Kontaktfläche von Grundplatte und Deckplatte ist dann entsprechend zu der konkaven sphärischen Fläche sphärisch konvex ausgebildet.

Die zuvor anhand der Figuren 3 bis 8 beschriebenen Ausführungsbeispiele sind insbesondere als Bandscheibenersatz geeignet. Durch gitterartige Ausbildung des rohrförmigen Abschnittes 100 kann der Operateur diesen rohrförmigen Abschnitt auf eine gewünschte Länge zuschneiden, beispielsweise auf die relativ kurze in Fig. 1 gezeigte Länge. Anschließend wird der so als Bandscheibenprotese ausgebildete Platzhalter zwischen zwei Wirbelkörper eingesetzt und greift mit den Zakken in die benachbarten Wirbelkörperendplatten ein, so daß die Platten selbst drehfest gehalten werden. Die elastischen Ringe bewirken eine Abfederung der Bandscheibenprotese gegen zu starkes Verkippen und bremsen gleichzeitig ein zu starkes Verdrehen um die Mittenachse 8. Die elastische Platte bewirkt jeweils eine Stoßdämpfung in axialer Richtung. Bei der Anwendung wird der Außendurchmesser von Grund- und Deckplatte so gewählt, daß er ein wenig kleiner ist als der kleinste Durchmesser der benachbarten Wirbelkörperendplattenflächen.

Ist der Platzhalter zum Ersatz eines oder mehrerer Wirbelkörper gedacht, dann wird, wie in Fig. 2 gezeigt, ein oben beschriebenes Element nicht nur von einer Seite, sondern auch von der anderen Seite jeweils bevorzugt im Paßsitz in den rohrförmigen Abschnitt 100' eingefügt, so daß die dadurch vorhandenen beiden Deckplatten jeweils um die Mittenachse des rohrförmigen Abschnittes 100' in oben beschriebener Weise kippbar ausgebildet sind.

Bei der Anwendung wird der so ausgebildete Platzhalter nach Entfernen des geschädigten Wirbelkörpers und der zugehörigen Bandscheiben zwischen die verbleibenden beiden Wirbelkörper eingesetzt und greift mit den Zacken in die benachbarten Wirbelkörperendplatten, so daß die gegenüberliegenden Deckplatten drehfest gehalten werden. Die Funktion der einzelnen Elemente wie Ring und planparallele Platten aus Kunststoff erfolgt wie oben beschrieben.

Gewünschtenfalls können die Kontaktflächen zwischen Deckplatte und Kern bzw. Grundplatte und Kern jeweils mit Materialien beschichtet werden, die eine besonders gute Gleitpaarung ergeben.

## Patentansprüche

1. Platzhalter für Wirbelkörper oder Bandscheiben mit einem rohrförmigen Abschnitt (100) und mit Zacken (103, 104) an den jeweiligen Enden des Platzhalters, wobei an einem Ende des Abschnittes ein Element (101) vorgesehen ist, welches eine Deckplatte (102) aufweist, die um einen Winkel zur Längsachse des Abschnittes kippbar ist, wobei der rohrförmige Abschnitt eine Mehrzahl von über die Oberflächen verteilt angeordneten Durchbrechungen bzw. Ausnehmungen aufweist.

2. Platzhalter nach Anspruch 1, wobei das Element eine der Deckplatte (72) gegenüberliegende Grundplatte (71) aufweist, wobei eine der Platten auf der anderen Platte zugewandten Seite eine konkave Kontaktfläche (77) und die andere Platte eine angrenzende konvexe Kontaktfläche (74) aufweisen.

3. Platzhalter nach Anspruch 2, wobei um eine der Kontaktflächen herum eine Rille vorgesehen ist, in der ein mit der gegenüberliegenden Kontaktfläche in Kontakt befindlicher elastischer Ring eingebettet ist.

4. Platzhalter nach Anspruch 3, wobei auch um die gegenüberliegende Kontaktfläche herum eine Rille vorgesehen ist, in die der Ring eingreift.

5. Platzhalter nach Anspruch 1, wobei das Element (101') eine der Deckplatte (72') gegenüberliegende Grundplatte (71') und einen dazwischenliegenden Kern (83) aufweist, wobei wenigstens eine der Platten auf der dem Kern zugewandten Seite eine erste konkave Kontaktfläche (77, 81) und der Kern wenigstens eine angrenzende erste konvexe Kontaktfläche aufweisen.

6. Platzhalter nach Anspruch 5, wobei um eine der Kontaktflächen herum eine Rille vorgesehen ist, in der ein mit der gegenüberliegenden Kontaktfläche in Kontakt befindlicher elastischer erster Ring eingebettet ist.

7. Platzhalter nach Anspruch 6, wobei auch um die gegenüberliegende Kontaktfläche herum eine Rille vorgesehen ist, in die der erste Ring eingreift.

8. Platzhalter nach Anspruch 6 oder 7, wobei auch um eine der zweiten Kontaktflächen herum eine Rille vorgesehen ist, in der ein mit der gegenüberliegenden Kontaktfläche in Kontakt befindlicher elastischer zweiter Ring eingebettet ist.

9. Platzhalter nach Anspruch 8, wobei auch um die mit dem zweiten Ring in Kontakt befindliche gegenüberliegende Kontaktfläche herum eine entsprechende Rille vorgesehen ist.

10. Platzhalter nach Anspruch 1, wobei das Element eine der Deckplatte (22) gegenüberliegende Grundplatte (21) und einen mit dieser in Kontakt befindlichen Kern (23) aufweist, der auf seiner der Grundplatte abgewandten Seite eine konvexe Oberfläche aufweist, wobei die Deckplatte auf der dem Kern zugewandten Seite einen konkav ausgebildeten Abschnitt (26) aufweist, und wobei der Kern eine der Grundplatte zugewandte elastische Schicht (29) und eine den konvexen Teil umfassende Gleitfläche umfaßt.

11. Platzhalter nach Anspruch 10, wobei auch die Grundplatte einen konkaven Abschnitt aufweist und der Kern angrenzend an die elastische Schicht eine mit dem konkaven Abschnitt in Eingriff befindliche konvexe Gleitfläche umfaßt.

12. Platzhalter nach Anspruch 11, wobei der Kern bikonvex ausgebildet ist und in seiner Mitte eine elastische Zwischenschicht besitzt.

13. Platzhalter nach einem der Ansprüche 10 bis 12, wobei entlang einer sich von der Grundplatte zur Deckplatte erstreckenden Mittenachse ein Dorn zum Begrenzen der Relativbewegung zwischen Grund- und Deckplatte um die Mittenachse herum vorgesehen ist.

14. Platzhalter nach einem der Ansprüche 1 bis 13, wobei jeweils die Kontaktflächen der Grund- bzw. Deckplatte konvex und die Kontaktflächen des Kerns konkav ausgebildet sind.

15. Platzhalter nach einem der Ansprüche 1 bis 14, wobei auch an dem anderen Ende des rohrförmigen Abschnittes (100') ein Element (101') nach einem der Ansprüche 2 bis 14 vorgesehen ist.

16. Platzhalter nach einem der Ansprüche 1 bis 15, wobei, die Ausnehmungen eine Mehrzahl von in Umfangsrichtung einander benachbarte rautenförmige Ausnehmungen umfassen.

## Claims

1. Spacer for vertebrae or intervertebral discs, with a tubular section (100), and with teeth (103, 104) at the respective ends of the spacer, wherein an element (101) is provided at one end of the section, which element (101) has a cover plate (102) that is tiltable about an angle to the longitudinal axis of the section, wherein the tubular section has a plurality of openings or recesses distributed over the surfaces.

2. Spacer according to Claim 1, wherein the element has a base plate (71) lying opposite the cover plate (72), wherein one of the plates, on the side facing the other plate, has a concave contact face (77), and the other plate has an adjoining convex contact face (74).

3. Spacer according to Claim 2, wherein a groove is provided around one of the contact faces, and an elastic ring situated in contact with the opposite contact face is embedded in the groove.

4. Spacer according to Claim 3, wherein a groove is also provided around the opposite contact face, in which groove the ring engages.

5. Spacer according to Claim 1, wherein the element (101') has a base plate (71') lying opposite the cover plate (72') and, between said plates, a core (83), wherein at least one of the plates, on the side facing the core, has a first concave contact face (77, 81), and the core has at least one adjoining first convex contact face.

6. Spacer according to Claim 5, wherein a groove is provided around one of the contact faces, and an elastic first ring situated in contact with the opposite contact face is embedded in the groove.

7. Spacer according to Claim 6, wherein a groove is also provided around the opposite contact face, in which groove the first ring engages.

8. Spacer according to Claim 6 or 7, wherein a groove is also provided around one of the second contact faces, and an elastic second ring situated in contact with the opposite contact face is embedded in the groove.

9. Spacer according to Claim 8, wherein a corresponding groove is also provided around the opposite contact face situated in contact with the second ring.

10. Spacer according to Claim 1, wherein the element has a base plate (21) lying opposite the cover plate (22) and has a core (23) which is situated in contact with the base plate (21) and which has a convex surface on its side directed away from the base plate, wherein the cover plate, on the side facing the core, has a concave section (26), and wherein the core comprises an elastic layer (29), facing the base plate, and a sliding surface that comprises the convex part.

11. Spacer according to Claim 10, wherein the base plate also has a concave section, and the core comprises, adjoining the elastic layer, a convex sliding surface situated in engagement with the concave section.

12. Spacer according to Claim 11, wherein the core is biconvex and has an elastic middle layer at its centre.

13. Spacer according to one of Claims 10 to 12, wherein, along a centre axis extending from the base plate to the cover plate, a projection is provided for limiting the relative movement between base plate and cover plate around the centre axis.

14. Spacer according to one of Claims 1 to 13, wherein the contact faces of the base plate and of the cover plate are each convex and the contact faces of the core are concave.

15. Spacer according to one of Claims 1 to 14, wherein an element (101') according to one of Claims 2 to 14 is also provided at the other end of the tubular section (100').

16. Spacer according to one of Claims 1 to 15, wherein the recesses comprise a plurality of diamondshaped recesses arranged adjacent to one another in the circumferential direction.

## Revendications

1. Ecarteur pour vertèbres ou disques intervertébraux, avec un tronçon (100) en forme de tube et avec des dentelures (103, 104) aux extrémités respectives de l'écarteur, dans lequel, à une extrémité du tronçon, est prévu un élément (101), présentant une plaque de couvercle (102), susceptible d'être basculée d'un certain angle par rapport à l'axe longitudinal du tronçon, le tronçon en forme de tube présentant une pluralité de passages ou évidements, répartis sur les surfaces.

2. Ecarteur selon la revendication 1, dans lequel l'élément présente une plaque de base (71) opposée à la plaque de couverture (72), l'une des plaques présentant, sur la face tournée vers l'autre plaque, une surface de contact concave (77) et l'autre plaque présentant une surface de contact convexe (74) limitrophe.

3. Ecarteur selon la revendication 2, dans lequel, autour d'une des surfaces de contact, est prévue une gorge dans laquelle est intégrée une bague élastique, se trouvant en contact avec la surface de contact opposée.

4. Ecarteur selon la revendication 3, dans lequel une gorge, dans laquelle s'engage la bague, est également prévue atour de la surface de contact opposée.

5. Ecarteur selon la revendication 1, dans lequel l'élément (101') présente une plaque de base (71'), opposée à la plaque de couverture (72'), et un noyau (83) intermédiaire, au moins l'une des plaques présentant, sur la face tournée vers le noyau, une première surface de contact concave (77, 81), et le noyau présentant au moins une première surface de contact convexe limitrophe.

6. Ecarteur selon la revendication 5, dans lequel, autour d'une des surfaces de contact, est prévue une gorge dans laquelle est intégrée une première bague élastique, se trouvant en contact avec la surface de contact opposée.

7. Ecarteur selon la revendication 6, dans lequel une gorge, dans laquelle s'engage la première bague, est également prévue atour de la surface de contact opposée.

8. Ecarteur selon la revendication 6 ou 7, dans lequel, autour d'une des deuxièmes surfaces de contact, est prévue une gorge dans laquelle est intégrée une deuxième bague élastique, se trouvant en contact avec la surface de contact opposée.

9. Ecarteur selon la revendication 8, dans lequel une gorge correspondante est également prévue atour de la surface de contact opposée, en contact avec la deuxième bague.

10. Ecarteur selon la revendication 1, dans lequel l'élément présente une plaque de base (21), opposée à la plaque de couverture (22), et un noyau (23), se trouvant en contact avec celle-ci, présentant une surface convexe sur sa face opposée à la plaque de base, la plaque de couverture présentant un tronçon concave (26) sur la face tournée vers le noyau, et le noyau comprenant une couche (29) élastique, tournée vers la plaque de base, et une surface de glissement entourant la partie convexe.

11. Ecarteur selon la revendication 10, dans lequel la plaque de base présente également un tronçon concave et le noyau comprenant, de manière limitrophe à la couche élastique, une surface de glissement convexe, se trouvant en prise avec le tronçon concave.

12. Ecarteur selon la revendication 11, dans lequel le noyau est configuré de manière biconvexe et comprend en son centre une couche intermédiaire élastique.

13. Ecarteur selon l'une des revendications 10 à 12, dans lequel un mandrin, pour limiter le déplacement relatif entre plaque de base et plaque de couverture autour de l'axe central, est prévu le long d'un axe central, s'étendant de la plaque de base à la plaque de couverture.

14. Ecarteur selon l'une des revendications 1 à 13, dans lequel les surfaces de la plaque de base ou de la plaque de couverture sont convexes, et les surfaces de contact du noyau sont concaves.

15. Ecarteur selon l'une des revendications 1 à 14, dans lequel un élément (101') selon l'une des revendications 2 à 14 est prévu à l'autre extrémité du tronçon (100') en forme de tube.

16. Ecarteur selon l'une des revendications 1 à 15, dans lequel les évidements comprennent une pluralité d'évidements en forme de losange, voisins les uns des autres en direction périphérique.
